# EUROPÄISCHE PATENTSCHRIFT

(11) **EP 0 813 904 B1**
(45) Veröffentlichungstag und Bekanntmachung des Hinweises auf die Patenterteilung: **15.09.2004**
(21) Anmeldenummer: 97109455.2
(22) Anmeldetag: 11.06.1997
(51) Int. Cl.: B01J 19/00, A61K 9/16, A61K 31/00

(54) **Verfahren zur Herstellung von Salzen von Säuregruppen tragenden pharmazeutischen Wirkstoffen**
Process for preparing salts of acid groups containing pharmaceutical actives
Procédé de préparation des sels d'agents pharmaceutiques contenant des groupes acides

(30) Priorität: 20.06.1996 DE 19624607
(43) Veröffentlichungstag der Anmeldung: 29.12.1997
(73) Patentinhaber: BASF AKTIENGESELLSCHAFT, 67056 Ludwigshafen (DE)
(72) Erfinder: Breitenbach, Jörg, Dr., 68199 Mannheim (DE); Rosenberg, Joerg, Dr., 67158 Ellerstadt (DE); Neumann, Jörg, Dr., 67117 Limburgerhof (DE); Zeidler, Jürgen, Dr., 67112 Mutterstadt (DE); Simon, Dirk, Dr., 67112 Mutterstadt (DE); Diener, Ralph, Dr., 67157 Wachenheim (DE)

(56) Entgegenhaltungen:
- EP-A- 0 238 240
- EP-A- 0 304 831
- WO-A-96/20155
- DE-A- 1 493 310

## Beschreibung

Die vorliegende Erfindung betrifft ein Verfahren zur Herstellung von Salzen von Säuregruppen tragenden pharmazeutischen Wirkstoffen.

Aus der EP-A 238 240 ist ein Verfahren bekannt, bei dem man die saure Form von Pflanzenschutz-Wirkstoffen mit einer Brönsted-Base kontinuierlich in einem Extruder zu Salzen der Wirkstoffe umsetzt. Als geeignete Basen werden Alkalimetallhydroxide, Erdalkalimetallhydroxide, Ammoniak und Amine genannt.

Die nach diesen bekannten Verfahren hergestellten Produkte zeigen jedoch noch eine merkliche Tendenz, aus der umgebenden Luft Feuchtigkeit aufzunehmen, zu verklumpen und zu zerfließen. Das führt in der Praxis zu dem Problem, daß die Qualität, vor allem die Fließfähigkeit dieser Produkte in angebrochenen Behältnissen ständig abnimmt.

Der vorliegenden Erfindung lag daher als Aufgabe ein Verfahren zur Herstellung von Salzen von Säuregruppen tragenden pharmazeutischen Wirkstoffen zugrunde, welches zu Produkten führt, welche diese nachteiligen Eigenschaften nicht aufweisen.

Demgemäß wurde ein Verfahren zur Herstellung von Salzen von Säuregruppen tragenden pharmazeutischen Wirkstoffen durch Umsetzung der Säuren mit einer Base in der Schmelze gefunden, welches dadurch gekennzeichnet ist, daß man die Säuren mit einer mindestens stöchiometrischen Menge einer Base im Extruder umsetzt.

Erfindungsgemäß werden als pharmazeutische Wirkstoffe solche Verbindungen eingesetzt, die eine Säuregruppe tragen, wobei es sich bevorzugt um Carbonsäuren oder Sulfonsäuren handelt.

Bevorzugt werden nach dem erfindungsgemäßen Verfahren Salze von Derivaten der Salicylsäure wie Acetylsalicylsäure oder von Arylcarbonsäuren wie beispielsweise Diclofenac, Tolmetidin oder Zomepirac hergestellt. Bevorzugte Carbonsäuren sind weiterhin Arylpropylcarbonsäurederivate wie Ibuprofen, Naproxen, Fenoprofen, Flurbiprofen oder Ketoprofen oder Arylessigsäurederivate wie Diclofenac oder auch Indol- und Indenessigsäurederivate wie Indometacin oder Sulindac. Weiterhin eignen sich Sulfonsäurederivate wie beispielsweise Metamizol.

Es können auch Wirkstoffgemische eingesetzt werden.

Anstelle der optisch aktiven Säuren können auch deren Racemate verwendet werden.

Weiterhin ist es möglich, die Carbonsäuren mit anderen Wirkstoffen zu kombinieren, beispielsweise mit Coffein oder Codein.

Als Basen eignen sich vor allem basische α-Aminocarbonsäuren, besonders bevorzugt Lysin. Definitionsgemäß sind selbstverständlich die als Basen verwendeten α-Aminocarbonsäuren nicht durch den Begriff "Säuregruppen tragende pharmazeutische Wirkstoffe umfaßt.

Weiterhin eignen sich als Basen Alkalimetall- oder Erdalkalimetallbasen.

Bevorzugte Alkalimetallbasen und Erdalkalimetallbasen sind Acetate, die bei der Umsetzung freie Essigsäure liefern, und Formiate, die in Wasser und Kohlenmonoxid zerfallen können, sowie vor allem Carbonate und Hydrogencarbonate (Bildung von Kohlendioxid) von Erdalkalimetallen und insbesondere von Alkalimetallen wie Natriumacetat, Kaliumacetat, Natriumformiat, Kaliumformiat, vorzugsweise Natriumcarbonat, Natriumhydrogencarbonat und Kaliumhydrogencarbonat, ganz besonders bevorzugt Kaliumcarbonat. Weiterhin eignen sich auch Gemische der vorstehend genannten Alkalimetallbasen und Erdalkalimetallbasen.

Die erfindungsgemäßen Basen werden mindestens in einer stöchiometrischen Menge, vorzugsweise im Überschuß vor allem in einem Überschuß von 1 bis 40 und ganz besonders bevorzugt von 2 bis 30 mol-%, bezogen auf die Säuren, eingesetzt.

Während bei einem Unterschuß der erfindungsgemäßen Basen in der Regel schmierige Produkte stark schwankender Konsistenz entstehen würden, erhält man bei Verwendung stöchiometrischer Mengen der erfindungsgemäßen Basen und ganz besonders bei einem Überschuß Produkte von granulärer Struktur mit ausgezeichneter Rieselfähigkeit und Lagerstabilität bei guter Löslichkeit und Auflösungsgeschwindigkeit in Wasser.

In den Ausgangsstoffen enthaltene und/oder bei der Umsetzung entstehende flüchtige Stoffe wie Wasser, Kohlendioxid usw. entweichen aus dem Produkt in der Regel während der Umsetzung oder bei hinreichend hoher Austrittstemperatur des Produktes aus dem Extruder auch noch danach.

Die Umsetzung im Extruder kann aber auch in Gegenwart eines Schleppmittels erfolgen, welches das Entweichen des Wassers unterstützt.

Geeignete Schleppmittel sind Cyclohexan, Toluol, Petrolether. Bevorzugt sind niedermolekulare Alkohole, insbesondere C₁-C₇-Alkanole und C₁-C₇-Alkandiole und ganz besonders bevorzugt primäre, sekundäre und tertiäre Alkohole wie Methanol, Ethanol, Propanol, Isopropanol, n-Butanol, iso-Butanol, tert.-Butanol, Pentanole, Hexanole, Heptanole und Mischungen hiervon.

In einer bevorzugten Ausführungsform des erfindungsgemäßen Verfahrens bringt man die aufgeschmolzene Carbonsäure im Extruder durch inniges Vermischen mit der festen erfindungsgemäßen Base zur Reaktion oder gibt die feste Säure in den Extruder, wo sie geschmolzen wird und mit der festen erfindungsgemäßen Base zur Reaktion gebracht wird.

Die erfindungsgemäßen Basen können auch gelöst oder suspendiert in dem Schleppmittel eingesetzt werden.

Feste Ausgangsstoffe können als solche oder in einem Lösungsmittel angeteigt, gelöst oder suspendiert dem Extruder zugeführt werden. Die Lösungsmittel sind so auszuwählen, daß ihre Verflüchtigung während der Herstellung der erfindungsgemäßen Extrudate gewährleistet ist. Vorzugsweise verwendet man als Lösungsmittel die vorstehend genannten Schleppmittel.

Die Ausgangsstoffe können vor der Umsetzung in Abhängigkeit von ihren physikalischen Eigenschaften zunächst vorgemischt werden. Eine Vorvermischung von flüssigen und festen Produkten etwa hat den Vorteil, daß eine Vorbenetzung der festen Stoffe erfolgt. Daraus resultieren im allgemeinen bei den Ausgangsstoffen günstigere Fließeigenschaften.

Kommt es bei der Vormischung zu Fließproblemen, so können Rieselhilfs- oder Antiblockmittel, z.B. Calciumcarbonat, Tricalciumphosphat, kolloidales Silicagel, mikrokristalline Cellulose, Stärke, Zuckeralkohole und/oder Talkum zum Einsatz kommen. Auch Farbstoffe können den Mischungen zugesetzt werden.

Weiterhin können mechanische Hilfsmittel wie Vibratoren und Rührer bei der Handhabung der Vormischung zum Einsatz kommen.

Auf die Art des Extruders kommt es bei dem erfindungsgemäßen Verfahren nicht an. Geeignet sind Einschneckenmaschinen, gegensinnig und gleichsinnig drehende, kämmende Schneckenmaschinen und Mehrwellenextruder. Diese Vorrichtungen sind dem Fachmann geläufig und bedürfen daher keiner weiteren Erläuterungen (vgl. etwa EP-A 238 240).

Bevorzugte Extruder sind gleichsinnig drehende, kämmende Schneckenmaschinen, besonders bevorzugt Doppelschneckenextruder.

Bei hygroskopischen oder reaktiven Ausgangsstoffen und/oder Produkten inertisiert man den Extruder vor der Umsetzung zweckmäßigerweise mit Stickstoff oder Kohlendioxid.

Die Umsetzung im Extruder und die anschließende Trocknung des Extrudats kann unter Normaldruck, im Unterdruck oder bei Überdruck bei Temperaturen von 0 bis 250, insbesondere von 60 bis 150°C durchgeführt werden. Die Reaktionszeit liegt im Bereich von 1 - 5 min.

Bei der Umsetzung im Extruder fällt das Produkt in Abhängigkeit von der Reaktionstemperaturführung bereits in Form von Partikeln oder als plastische Masse an, welche in an sich bekannter Weise ausgeformt werden kann.

Vorzugsweise arbeitet man so, daß Partikel von 0,1 bis 5, vorzugsweise von 0,1 bis 3 cm mittlerem Durchmesser erhalten werden.

Die erfindungsgemäß hergestellten Extrudate können im übrigen, soweit ihnen noch flüchtige Stoffe anhaften, von diesen nach herkömmlichen (Trocknungs-)Verfahren befreit werden.

Die nach dem erfindungsgemäßen Verfahren hergestellten Extrudate eignen sich zur Direkttablettierung, Granulierung oder Pelletierung. Sie können auch in einem anschließenden Schmelzextrusionsverfahren mit üblichen pharmazeutischen Hilfsstoffen verarbeitet werden.

### Herstellbeispiele

Für die Herstellung der Extrudate wurde eine gleichsinnig drehende, kämmende Doppelschneckenextruder (ZSK 30 der Firma Werner & Pfleiderer, Stuttgart, Deutschland) verwendet, welche aus 8 separat heiz- bzw. kühlbaren kammerartigen Zonen bestand. Im folgenden werden die Zonen als "Zone 1", "Zone 2" usw. bezeichnet, wobei die Ausgangsstoffe bei Zone 1 eintreten und bei Zone 8 austreten sollen. Zone 1 wurde mit Wasser gekühlt (Temperatur des ablaufenden Wassers: 38°C). Die Zone 2 wurde während der gesamten Versuchsdauer bei 80°C, die Zonen 3 und 4 bei 128°C betrieben. Die Zone 4 war ferner nach oben offen, um bei der Rektion gebildetes Kohlendioxid und Wasserdampf ausgasen zu lassen. Die Zonen 5, 6, 7 bzw. 8 wurden mit Wasser gekühlt, wobei sich Kühlwasserablauftemperaturen von 28°C, 21°C, 21°C bzw. 20°C ergaben.

### Beispiel 1

### Herstellung des Natriumsalzes eines Ibuprofenracemats

In Zone 1 dieses Extruders wurden bei einer Umdrehungszahl von 36 U/min. stündlich 484 g (3,5 Mol) Natriumcarbonat und 1238 g (6 Mol) Ibuprofen über mit Schnecken ausgerüstete Dosierbandwagen eingetragen. Der Extruder wurde 40 Stunden betrieben. Das Ibuprofen wurde bei diesem Versuch vollständig zum Natriumsalz umgesetzt.

Bei dem auf entsprechende Weise erhaltenen Extrudat, handelte es sich um ein grobkörniges, gut wasserlösliches Granulat.

200 mg des Extrudats (Korngröße 0,5 - 2 mm) wurden in eine Wirkstofffreisetzungsapparatur gemäß USPXXII ("Paddle"-Apparatur) gegeben. Innerhalb von 30 min. wurde der Wirkstoff vollständig freigesetzt und es bildete sich eine klare Lösung.

## Patentansprüche

1. Verfahren zur Herstellung von Salzen von Säuregruppen tragenden pharmazeutischen Wirkstoffen durch Umsetzung der Carbonsäuren mit einer Base in der Schmelze, **dadurch gekennzeichnet, daß** man die Säuren mit einer mindestens stöchiometrischen Menge einer Base im Extruder umsetzt.

2. Verfahren nach Anspruch 1, **dadurch gekennzeichnet, daß** man als Base eine basische Aminosäure einsetzt.

3. Verfahren nach Anspruch 2, **dadurch gekennzeichnet, daß** man als Base Lysin einsetzt.

4. Verfahren nach Anspruch 1, **dadurch gekennzeichnet, daß** man als Base eine Alkali- oder Erdalkalimetallbase einsetzt.

5. Verfahren nach Anspruch 4, **dadurch gekennzeichnet, daß** man eine Alkali- oder Erdalkalimetallbase, welche unter den Reaktionsbedingungen teilweise oder vollständig unter Bildung flüchtiger Stoffe zerfällt, einsetzt.

6. Verfahren nach Anspruch 5, **dadurch gekennzeichnet, daß** man Alkali- oder Erdalkalicarbonate einsetzt.

## Claims

1. A process for preparing salts of pharmaceutical active substances which have acidic groups by reacting the carboxylic acids with a base in the melt, wherein the acids are reacted with at least the stoichiometric amount of a base in an extruder.

2. A process as claimed in claim 1, wherein a basic amino acid is employed as base.

3. A process as claimed in claim 2, wherein lysine is employed as base.

4. A process as claimed in claim 1, wherein an alkali metal or alkaline earth metal base is employed as base.

5. A process as claimed in claim 4, wherein an alkali metal or alkaline earth metal base which, under the reaction conditions, decomposes partly or completely to form volatile substances is employed.

6. A process as claimed in claim 5, wherein alkali metal or alkaline earth metal carbonates are employed.

## Revendications

1. Procédé pour la préparation de sels de substances à activité pharmaceutique portant des groupes acides par réaction des acides carboxyliques avec une base à l'état fondu, **caractérisé par le fait qu'**on fait réagir les acides avec une quantité au moins stoechiométrique d'une base dans une extrudeuse.

2. Procédé selon la revendication 1, **caractérisé par le fait qu'**on utilise comme base un acide aminé basique.

3. Procédé selon la revendication 2, **caractérisé par le fait qu'**on utilise comme base la lysine.

4. Procédé selon la revendication 1, **caractérisé par le fait qu'**on utilise comme base une base de métal alcalin ou de métal alcalino-terreux.

5. Procédé selon la revendication 4, **caractérisé par le fait qu'**on utilise une base de métal alcalin ou de métal alcalino-terreux qui se décompose partiellement ou totalement dans les conditions de la réaction, avec formation de substances volatiles.

6. Procédé selon la revendication 5, **caractérisé par le fait qu'**on utilise des carbonates alcalins ou alcalino-terreux.
